# EUROPEAN PATENT APPLICATION

(11) **EP 2 275 064 A1**
(43) Date of publication of application: **19.01.2011**
(21) Application number: 09727930.1
(22) Date of filing: 31.03.2009
(51) Int. Cl.: A61F 13/15, A61F 13/49, A61F 13/56

(54) **MANUFACTURING METHOD FOR ABSORBENT PRODUCTS AND MANUFACTURING DEVICE FOR ABSORBENT PRODUCTS**

(30) Priority: 31.03.2008 JP 2008094064; 25.03.2009 JP 2009074773
(71) Applicant: Unicharm Corporation, Ehime 799-0111 (JP)
(72) Inventor: SAKAGUCHI, Satoru, Kagawa 769-1602 (JP); KAMEDA, Noritomo, Kagawa 769-1602 (JP); OKU, Tomomi, Kagawa 769-1602 (JP)
(74) Representative: Eke, Philippa Dianne
(86) International application number: PCT/JP2009/056622
(87) International publication number: WO 2009/123178

(57) **Abstract**

Provided is a manufacturing method for absorbent products that has a fastening member placement step (S21) wherein latch members (40a, 40b), which are fastenable to prescribed areas, are placed on both sides of a center line (C) that is parallel to the conveyance direction of the flap line on a flap continuum (130) that are continuously conveyed on the flap line, a flap formation process (S23) wherein flaps (30) are formed by cutting the flap continuum (130) on the flap line in a direction that intersects with the conveyance direction of the flap line, and a flap placement step (S33) wherein flaps are placed on front waistline member continuum or the rear waistline member continuum (120a) or (120b) on the main body line so that the center line (C) is orthogonal to the conveyance direction of the main body line, and both edges (50a, 50b) of the flap continuum (130) on the flap line are folded over toward the center line (C).

## Description

### TECHNICAL FIELD

The present invention relates to a manufacturing method and a manufacturing apparatus for absorbent articles each of which includes: a front waistline member, a rear waistline member and a crotch member that connects the front waistline member and the rear waistline member.

### BACKGROUND ART

In general, in order to be easily put on the wearer, an open-type diaper needs to be provided with: a vertically long absorber (absorber body) that absorbs body fluid from the body of the wearer, a diaper main body to which the absorber is attached, side flaps (hereinbelow referred to as flap) that project outwardly in the width direction of the absorber from the absorber body.

Heretofore, for providing such side flaps, a following method has been known; that is, forming side panel members projecting outwardly from an absorbent body, and thereafter trimming the side panel members so that remaining parts thereof can serve as side flaps. However, this method has a problem in which many materials go wasted because of the trimming.

As a method for solving this problem, a manufacturing method for a diaper disclosed in Patent Literature 1 is known.

In this method for manufacturing the diapers, following steps are included:
(1) a step of placing absorbers while being spaced apart from one another, in an MD direction (longitudinal direction of a web) on the web having a first side edge portion and a second side edge portion; and
(2) a step of cutting the web in a CD direction (width direction of the web) so that the web is divided into a first group and a second group and each of the first group and the second group includes the absorbers, the first group including the first side edge portion to be served as horizontally long portions of the diapers (corresponds to side flaps), and the second group including the second side edge portion to be served as horizontally long portions of the diapers. Patent Literature 1: Japanese Patent Application Publication No. 2003-102777

However, the above-described manufacturing method for a diaper has a problem in which the form of manufactured diapers is limited to substantially a T-letter shape.

In addition, in the above-described manufacturing method for the diapers, a long web that is continuously transported is alternately cut into nested shapes. Accordingly, after being cut, the diapers are transported with their horizontal portions unconnected. Thus, the method has a problem that the diapers are transported unstably.

In addition, the method has a problem in which extremely complex step is required when a step of folding-back is performed on the diapers that is transported in the above manner.

The present invention has been made in view of the foregoing problems, and has an object of providing a manufacturing method and a manufacturing apparatus for absorbent articles being able to realize a stable transportation, and to reduce a material loss in forming side flaps without requiring any complex step.

### DISCLOSURE OF THE INVENTION

An aspect of the present invention is summarized as a manufacturing method for absorbent articles each including: front waistline members, rear waistline members, and crotch members, the crotch members connecting each of the front waistline members and each of the rear waistline members. The manufacturing method for the absorbent articles according to the present invention includes: placing latch members that can be attached to and locked in predetermined regions, on both sides of a vicinal region of a center line extending along a moving direction of a flap line, on a flap continuum having a long shape and being continuously transported on the flap line; forming flaps on the flap line, by cutting the flap continuum in a direction orthogonal to the moving direction of the flap line; and placing each of the flaps, on a main body line, on a front waistline member continuum or on a rear waistline member continuum, so that the center line is orthogonal to a moving direction of the main body line. On the flap line, both side edge portions of the flap continuum are folded back toward the center line.

As described above, the present invention can provide a manufacturing method and a manufacturing apparatus for absorbent articles being able to realize a stable transportation and to reduce a material loss in forming side flaps without requiring any complex step.

### BRIEF DESCRIPTION OF DRAWINGS

[Fig. 1] Fig. 1 is a perspective view showing an overall structure of an absorbent article according to a first embodiment of the present invention.
[Fig. 2] Fig. 2 is a plan view showing the absorbent article according to the first embodiment of the present invention.
[Fig. 3] Fig. 3 is a plan view showing the developed absorbent article according to the first embodiment of the present invention.
[Fig. 4] Fig. 4 is a view for illustrating a manufacturing method for an absorbent article according to the first embodiment of the present invention.
[Fig. 5] Fig. 5 is a plan view showing an absorbent article according to a second embodiment of the present invention.
[Fig. 6] Fig. 6 is a plan view showing the developed absorbent article according to the second embodiment of the present invention.
[Fig. 7] Fig. 7 is a view for illustrating a manufacturing method for an absorbent article according to the second embodiment of the present invention.
[Fig. 8] Fig. 8 is a perspective view of a latch member placing structure 210 according to a third embodiment of the present invention.
[Fig. 9] Fig. 9 is a perspective view of a cutting and placing structure 230 according to a modified example of the third embodiment of the present invention.
[Fig. 10] Fig. 10 is a perspective view of a cutting and placing structure 230 according to the third embodiment of the present invention.
[Fig. 11] Fig. 11 is a perspective view of a cutting and placing structure 250 according to the third embodiment of the present invention.

### PREFERRED EMBODIMENTS FOR CARRYING OUT THE INVENTION

### [First Embodiment]

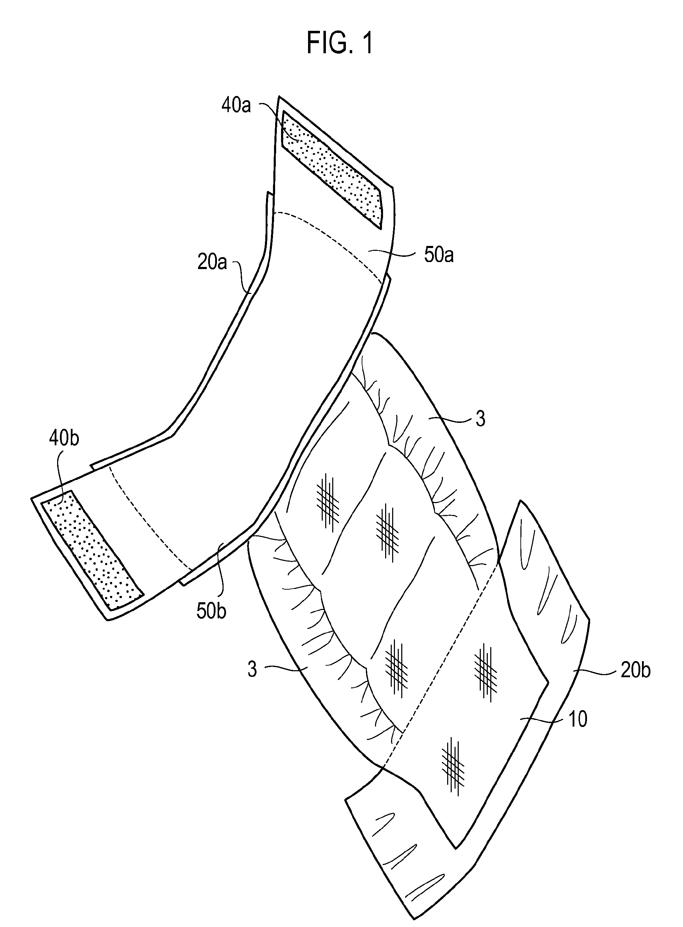

Referring to Fig. 1 to Fig. 4, a manufacturing method for absorbent articles according to a first embodiment of the present invention will be described.

According to the manufacturing method for absorbent articles of the first embodiment, absorbent articles (see, Fig. 1 to Fig. 3) each of which includes a front waistline member 20b, a rear waistline member 20a, and a crotch member (absorber body) 10 connecting the front waistline member 20b and the rear waistline member 20a can be manufactured.

Here, Fig. 1 is a perspective view showing an overall structure of an open-type diaper (absorbent article) manufactured by the manufacturing method for absorbent articles according to the present embodiment.

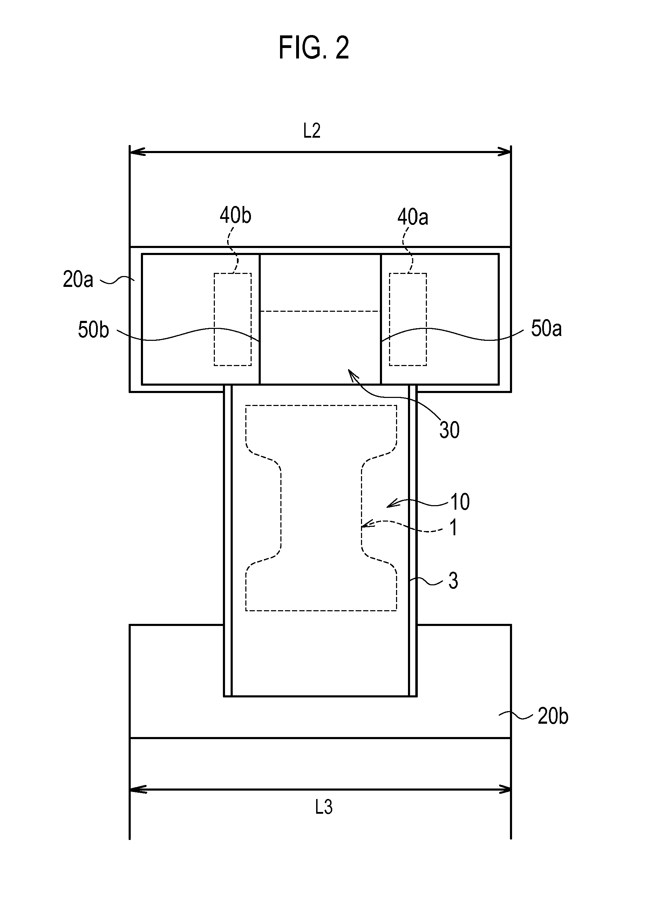

Fig. 2 is a view showing a state where latch members 40a and 40b are locked with and temperately bonded to a flap body 30, in the open-type diaper manufactured by the manufacturing method for absorbent articles according to the present embodiment.

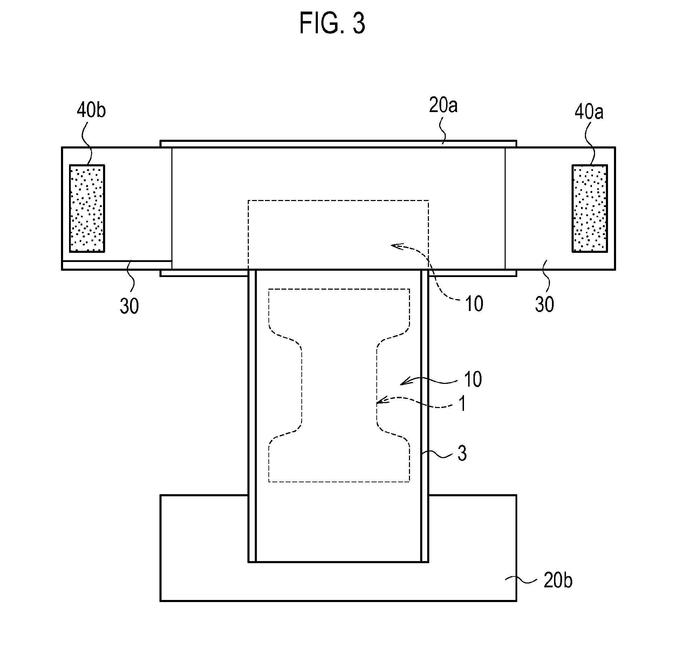

Fig. 3 is a view showing a state where the flap 30 (folded back portions 51 a and 51 b) is open in the open-type diaper manufactured by the manufacturing method for absorbent articles according to the present embodiment.

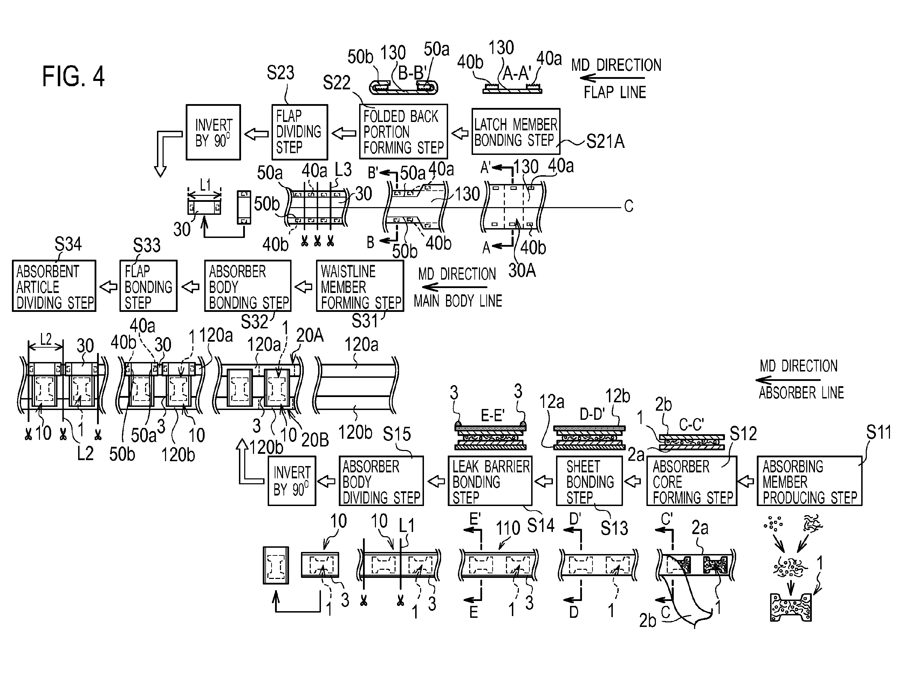

The manufacturing method for the absorbent article according to the present embodiment will be described below by referring to Fig. 4.

As shown in Fig. 4, in the manufacturing method for absorbent articles according to the present embodiment, following lines are used: a flap line, an absorber line, and a main body line. The flap line is used for transporting a long continuum of the flaps (flap continuum 130), the absorber line is used for transporting a long continuum of the crotch members (crotch member continuum 110), and the main body line is used for transporting a pair of continuums of the waistline members (that is, a front waistline member continuum 120a and a rear waistline member continuum 120b) both having a long shape.

Note that, as shown in Fig. 4, the present embodiment is described for an example in which an MD direction (moving direction) of the flap line, an MD direction of the main body line, an MD direction of the absorber line are same. However, the present invention is not limited to this example.

Firstly, steps performed on the absorber line on which the absorbers to be placed on the crotch members 10 are transported will be described.

In step S11, a crusher crushes a pulp sheets to produce crushed pulps. Thereafter, when the crushed pulps pass through a pulp supply duct, a super absorbent polymer (SAP) is mixed thereto.

Then, the mixture of the crushed pulp and the super absorbent polymer, which is supplied from the pulp supply duct, is layered in a predetermined shape by use of a layer drum.

In step S12, on sheet tissues 2a being continuously transported in the MD direction of the absorber line, the absorber cores 1 are placed by a predetermined interval length in the MD direction (moving direction) of the absorber line.

Subsequently, on the sheet tissues 2a, sheet tissues 2b being continuously transported in the MD direction of the absorber line is layered. Here, on the sheet tissues 2a, a plurality of absorber cores 1 is placed along the MD line of the absorber line.

In step S13, top sheets (liquid permeable) 12b being continuously transported in the MD direction of the absorber line are placed on and bonded with the sheet tissues 2b. In addition, back sheets (liquid impermeable) 12a being continuously transported in the MD direction of the absorber line are placed under and bonded with the sheet tissues 2a.

In step S14, leak barriers 3 formed of different members are bonded to both side edges of each of the crotch member 10 in the MD direction of the absorber line.

In step S15, the crotch member continuum 110 (absorber body) that is continuously transported in the MD direction of the absorber line is transported by use of a rotation of a rotating drum. Concurrently, the crotch member continuum 110 is cut in the CD direction by a predetermined interval length, by use of a cutter roll arranged opposite to the outer peripheral surface of the rotating drum. Thereby the crotch members 10 are formed.

Secondly, steps performed on the flap line on which the flaps 30 to be placed on the rear waistline members 20a are transported will be described.

In step S21, on the flap continuum 130 having a long shape and being continuously transported on the flap line, latch members 40a and 40b that can be attached to and locked in predetermined regions of the front waistline members 20b are placed on both sides of the center line C that extends along the MD direction of the flap line.

Specifically, the latch members 40a and 40b are bonded to both side edge portions 50a and 50b of the flap continuum 130, by the predetermined interval length.

In this regard, when hook members (male members) are placed as the latch members 40a and 40b, hooked members (female members) are provided as latched members in the predetermined regions of the front waistline members 20b.

Note that, in the present embodiment, the front waistline members 20b are made of a non-woven fabric. Accordingly, the predetermined regions of the front waistline members 20b themselves can serve as the hooked members even when the hooked members are not additionally provided.

In step S22, on the flap line, both of the side edge portions 50a and 50b of the flap continuum 130 are folded back toward the center line C. Concurrently, the latch members 40a and 40b that are respectively placed on the both of the side edge portions 50a and 50b are locked with and temporarily bonded to the flap continuum 130.

Here, after both of the side edge portions 50a and 50b are locked with the flap continuum 130 by the latch members 40a and 40b, an embossing step or the like may be further performed so as to temporarily bond the both of the edge portions 50a and 50b with the flap continuum 130.

Note that, as will be described later, both of the side edge portions 50a and 50b of the flap continuum 130 are folded back toward the center line C so that each width of the flaps 30 in the direction crossing the center line C becomes shorter than each of the cut lengths of the front waistline members 20b and the rear waistline members 20a.

In step S23, on the flap line, the flap continuum 130 is cut by the predetermined interval length in the direction (CD direction) crossing the MD direction of the flap line. Thereby, the flaps 30 are formed.

In this regard, before the flap continuum 130 is cut, cut-out portions, each of which has a predetermined shape (for example, a curved shape along a leg), may be formed in the part of both of the side edge portions 50a and 50b, which are temporarily bonded to the flap continuum 130.

Alternatively, after the flap continuum 130 is cut, cut-out portions, each of which has a predetermined shape (for example, a curved-shape along a leg), may be formed in the part of both of the edge portions 50a and 50b, which are temporarily bonded to the flaps 30.

Thirdly, steps performed on the main body line on which the front waistline member continuum 120b and the rear waistline member continuum 120a are transported will be described.

In step S31, a pair of continuums of waistline members (the front waistline member continuum 120b and the rear waistline member continuum 120a) both having a long shape is produced from non-woven fabric and transported in the MD direction of the main body line.

Note that, the front waistline member continuum 120b and the rear waistline member continuum 120a may have stretching properties.

In step S32, the crotch members 10 formed in step S15 on the absorber line is placed while being spaced apart from one another in the MD direction of the main body line, between the front waistline member continuum 120b having a long shape and the rear waistline member continuum 120a having a long shape. Here, the front waistline member continuum 120b of and the rear waistline member continuum 120a are continuously transported on the main body line.

In step S33, on the main body line, the flaps 30 each formed in step S23 is placed on the rear waistline member continuum 120a so that the center line C of each of the flaps 30 can be orthogonal to the MD direction of the main body line.

Specifically, in step S33, on the main body line, the flaps 30 each formed in step S23 are bonded to the rear waistline member continuum 120a so that the flaps 30 can be respectively placed on the region 20A each constituting the absorbent articles (rear waistline members 20a), on the rear waistline member continuum 120a.

In addition, the flaps 30 each formed in step S23 on the flap line are rotated by 90°, and thereafter is bonded on a thermoplastic resin on the rear waistline member continuum 120a.

In this regard, the thermoplastic resin may be entirely applied onto the back side of each of the flaps 30, or the thermoplastic resin may be applied onto only a part of the back side of the each of the flaps 30.

Further, in step S33, the flaps 30 may be bonded on the rear waistline member continuum 120a by use of a thermo compression or the like, using an embossing roller or ultrasonic waves. In addition, in step S33, each of the flaps 30 may be placed so as to cover an edge portion of each of the crotch members 10 on the rear waistline member continuum 120a.

In step S34, on the main body line, the front waistline member continuum 120b and the rear waistline member continuum 120a are cut in the CD direction of the main body line by a predetermined interval length.

Specifically, the front waistline member continuum 120b and the rear waistline member continuum 120b are cut so that each lengths L3 of each of the front waistline members 20b in the width direction of the absorbent article and each lengths L2 of each of the rear waistline members 20a in the width direction of the absorbent article can be equal.

Here, a width L1 in the direction crossing the center line C of the flaps 30 is shorter than a predetermined length (pitch) L2 being the cut length of the front waistline member continuum 120b and the rear waistline member continuum 120a.

Note that, in the above-described example, description has been given of the example in which the flaps 30, on which the latch members 40a and 40b that can be attached to and locked in the predetermined regions of the front waistline member 20b are placed, are placed on the rear waistline member continuum 120a. However, the present invention is not limited to this example but can be applied to an example in which the flaps 30, on which the latch members 40a and 40b that can be attached to and locked in the predetermined regions of the front waistline members 20b are placed, are placed on the front waistline member continuum 120b.

According to the manufacturing method for absorbent articles of the first embodiment of the present invention, absorbent articles having one pair of side flaps protruded from the absorber body can be manufactured without limitation in the product forms.

According to the manufacturing method for the absorbent articles of the first embodiment of the present invention, the crotch members 10 can be continuously transported on the main body line. Therefore, a stable transportation can be realized and the absorbent articles can be folded back without requiring any complex step.

According to the manufacturing method for the absorbent articles of the first embodiment of the present invention, each of the flaps 30 is locked with the latch members 40a and 40b so that each of the absorbent articles can be prevented from being turned over while being transported.

According to the manufacturing method for the absorbent articles of the first embodiment of the present invention, the width L1 in the direction crossing the center line C of each of the flaps 30 is shorter than the predetermined length (pitch) L2. Accordingly, both of the edge portions 50a and 50b of the flaps 30 can be prevented from being cut caused by the misalignment in the cutting process on the main body line.

According to the manufacturing method for the absorbent articles of the first embodiment of the present invention, the front waistline member continuum 120b and the rear waistline member continuum 120a are continuously transported. Accordingly, the edge portion of the front waistline member continuum 120b and the edge portion of the rear waistline member continuum 120a can be easily folded back and stacked. Therefore, the front waistline member continuum 120b and the rear waistline member continuum 120a thus stacked can be simultaneously cut. Thus, open-type diapers and pants-type diapers can be manufactured in the same cutting step. Therefore, the open-type diapers and the pants-type diapers can be manufactured without changing the cutting step or a cutter itself.

According to the manufacturing method for the absorbent articles of the first embodiment of the present invention, each of the flaps 30 is placed so as to cover the edge portion of the crotch members 10 on the rear waistline member continuum 120a. Accordingly, it is possible to prevent leakage of the super absorbent polymer, which forms the absorber cores 1.

According to the manufacturing method for the absorbent articles of the first embodiment of the present invention, portions other than the edge portions 50a and 50b of the flaps 30 can be set to be a region bonded to the rear waistline members 20a. Thus, a bonding strength between the flaps 30 and the rear waistline members 20a can be kept at a high level. Furthermore, leakage of the thermoplastic rein, which occurs when the bonding region is small, can be solved.

### [Second Embodiment]

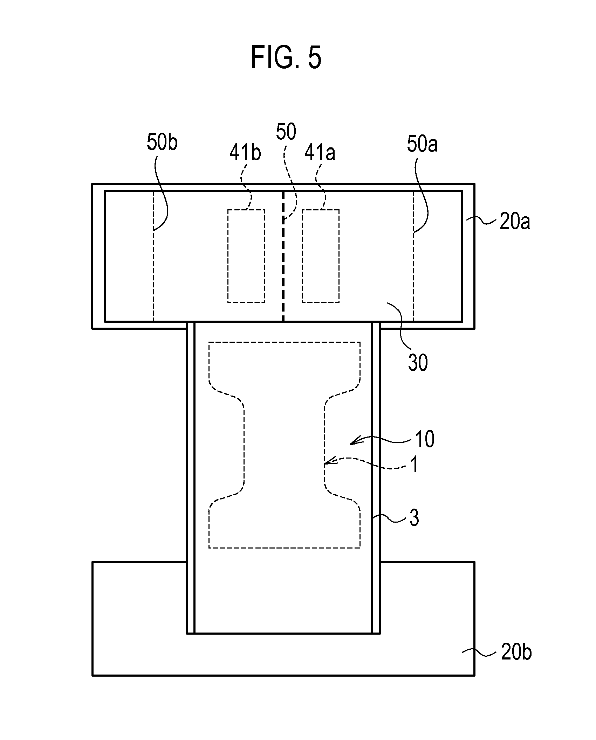

Referring to Fig. 5 to Fig. 7, a manufacturing method for absorbent articles according to a second embodiment of the present invention will be described. In the following description, the manufacturing method for the absorbent articles according to the second embodiment of the present invention will be described by focusing mainly on the differences with the manufacturing method for the absorbent articles according to the first embodiment of the present invention.

Fig. 5 is a view showing a state where latch members 41 a and 41 b are locked with and temporarily bonded to a rear waistline member 20a, in an open-type diaper manufactured by the manufacturing method for an absorbent article according to the present embodiment.

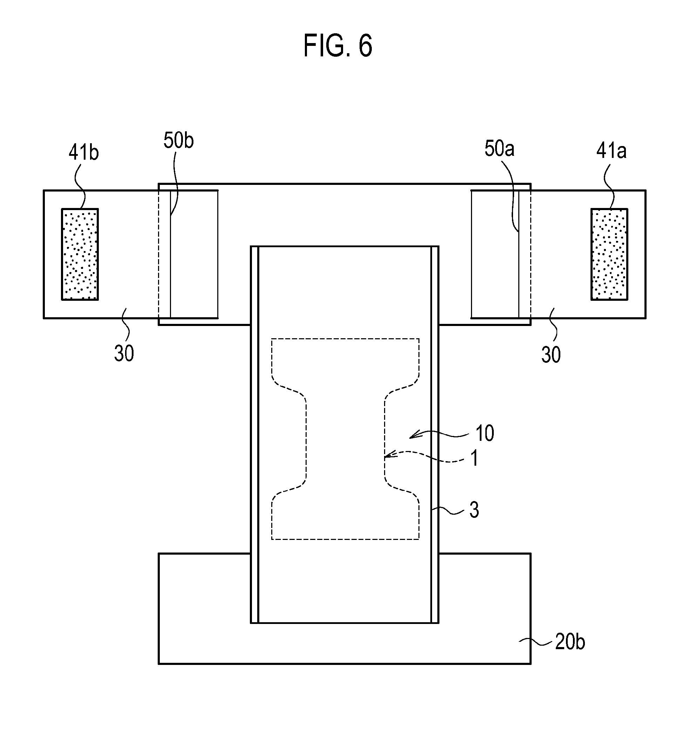

Fig. 6 is a view showing a state where a potential cut line (perforated line) 50 to be described later is torn and a flap 30 is open in the open-type diaper manufactured by the manufacturing method for an absorbent article according to the present embodiment.

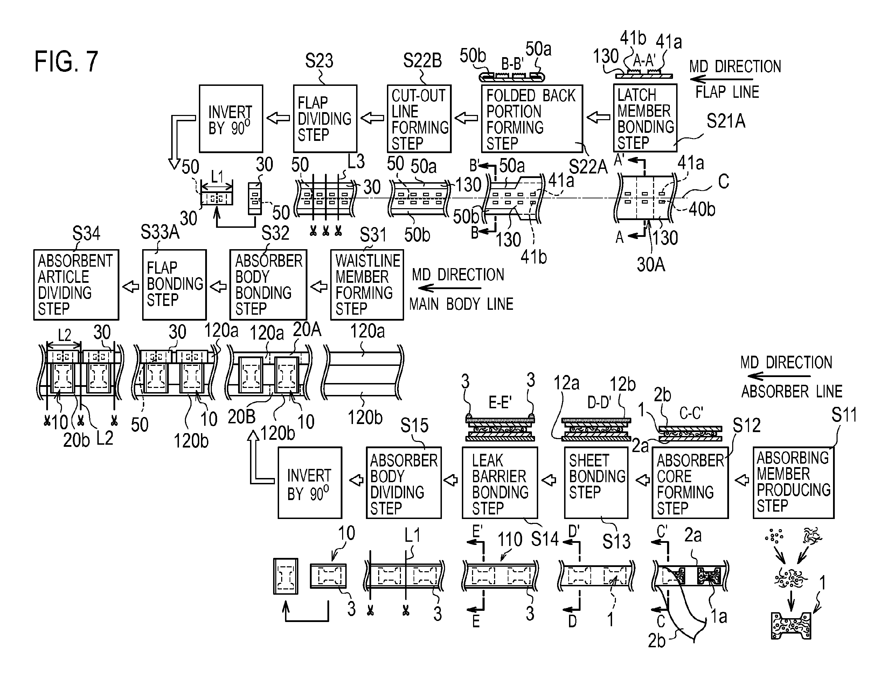

The manufacturing method for the absorbent articles according to present invention will be described below by referring to Fig. 7.

Firstly, steps performed on an absorber line are same as those performed on the absorber line in the manufacturing method for an absorbent article according to the above-described first embodiment. Accordingly, the description thereof will be omitted.

Secondly, steps performed on a flap line will be described.

In step S21A, on a flap continuum 130 having a long shape and being continuously transported on the flap line, latch members 41 a and 41 b that can be attached to and locked in a predetermined region of the front waistline members 20b are placed on both sides of the center line C that extends along the MD direction of the flap line.

Specifically, the latch members 41 a and 41 b are placed on the flap continuum 130 by a predetermined interval length, on both sides of a vicinal region of the center line C that extends along the MD direction of the flap line.

In addition, one latch member may be placed across both sides of the center line C instead of respectively placing the two latch members 41 a and 41 b on both sides of the center line C.

In step S22A, on the flap line, edge portions 50a and 50b of both sides of the flap continuum 130 are folded back toward the center line C. Specifically, as shown in the cross-sectional view taken along B-B' line, on the flap line, both of the edge portions 50a and 50b of the flap continuum 130 are folded back on the upper side of the flap continuum 130.

In this regard, in order to prevent the flap continuum 130 from being turned over while being transported, an embossing step or the like may be performed on folded edge portions 51 a and 51 b of the flaps 30 so that the folded edge portions 51 a and 51 b can be temporarily bonded to the flap continuum 130. Here, the folded back portions 51 a and 51 b are referred to as portions formed by the side edge portions 50a and 50b being folded back to cause the flaps 30 to overlap.

In step S22B, on the flap continuum 130, a potential cut line (perforated line) 50 that halves the flap continuum 130 is formed.

Note that, the potential cut line 50 may be formed in step S21A, S22A or S23. In addition, the potential cut line 50 may be straight or curved as long as it extends along the center line C.

In addition, in any one of the steps performed on the flap line, a cut-out portion having a predetermined shape (for example, curved-shape along a leg) may be formed in a part of the vicinal region of the center line C.

Thirdly, steps performed on a main body line will be described. Note that, steps S31 and S32 are same as steps S31 and S32 in the manufacturing method for an absorbent article according the above-described first embodiment, and the description thereof will be omitted.

In step S33A, on the main body line, the flaps 30 each formed in step S23 is placed on the rear waistline member continuum 120a so that the center line C of the flaps 30 can be orthogonal to the MD direction of the main body line.

Specifically, on the flap line, each of the flaps 30 formed in step S23 is rotated by 90° and inverted vertically. Thereafter, each of the flaps 30 is bonded to the rear waistline member continuum 120a by use of a thermoplastic resin.

Specifically, in step S33A, on the main body line, each of the flaps 30 is bonded to the rear waistline member continuum 120a so that the flaps 30 can be respectively placed on the regions 20A each constituting the absorbent articles (rear waistline members 20) on the rear waistline member continuum 120a.

In this regard, in step S33, it is preferable that the flaps 30 be bonded onto the rear waistline member continuum 120a by use of a thermo compression or the like, using an embossing roller or ultrasonic waves. Furthermore, the thermoplastic resin may be entirely applied onto the back side of the folded edge portions 51 a and 51 b of the flaps 30, the entire back surface corresponding to the folded back portions 51 a and 51 b, or the thermoplastic resin may be applied onto only a part of the back side of the folded back portions 51 a and 51 b of the flaps 30, the part of the back surface corresponding to the folded back portions 51 a and 51 b.

In step S34, on the main body line, the front waistline member continuum 120b and the rear waistline member continuum 120a are cut in the CD direction of the main body line by a predetermined interval length.

Specifically, the front waistline member continuum 120b and the rear waistline member continuum 120a are cut so that each lengths L3 of the front waistline members 20b in the width direction of the absorbent article and each lengths L2 of the rear waistline members 20a in the width direction of the absorbent article can be equal.

Here, the width L1 in the direction crossing the center line C of the flaps 30 is shorter than a predetermined length (pitch) L2 that is the cut length of the front waistline member continuum 120b and the rear waistline member continuum 120a.

According to the manufacturing method for the absorbent articles of the second embodiment of the present invention, the potential cut line 50 is formed along the center line of the flaps 30, and the flaps 30 are not divided into two parts. Therefore, on the main body line, it is possible to prevent the flaps 30 from being turned over while being transported.

### [Third Embodiment]

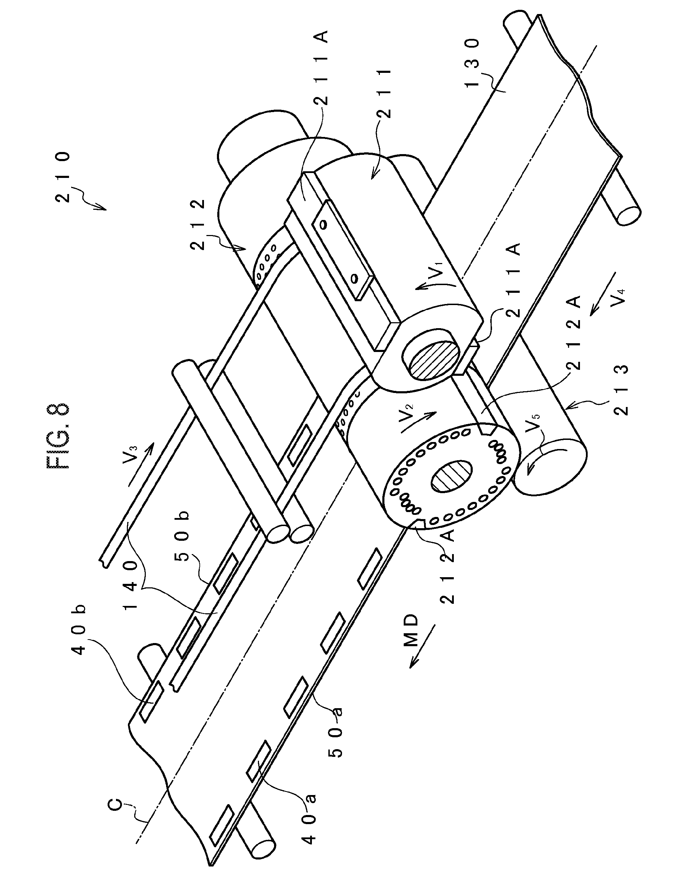

Hereinbelow, a manufacturing method for absorbent articles according to the third embodiment of the present invention will be described by referring to Figs. 8 to 10. The manufacturing method for the absorbent articles according to the third embodiment is provided with: a latch member placing structure 210; a side edge portion folding back structure 220; and a cutting and placing structure 230.

By referring to Figures, there will be described a latch member placing structure 210 according to the third embodiment of the present invention. Fig. 8 is a perspective view of a latch member placing structure 210 according to a third embodiment of the present invention.

As shown in Fig. 8, on the flap continuum 130 having a long shape and being transported continuously on the flap line (above-described step S21), the latch member placing structure 210 places the latch members 42a and 42b by a predetermined interval length, on both sides of the vicinal region of the center line C that extends along the MD direction of the flap line. In the present embodiment, the latch member placing structure 210 places the latch members 42a and 42n on both of the side edge portions 50a and 50b of the flap continuum 130.

The latch member placing structure 210 is mainly provided with: an upper blade roll 211, a lower blade roll 212, and a transcript roll 213. Note that, the upper blade roll 211, the lower blade roll 212, and the transcript roll 213 are configured to rotate by using a shaft (not shown) as a center, respectively.

The upper blade roll 211 includes two blades 211 A for cutting the latch member continuum 140. The circumferential velocity (V₁) of the upper blade roll 211 is substantially same as the circumferential velocity (V₂) of the lower blade roll 212.

In addition, the circumferential velocity (V₁) of the upper blade roll 211 is faster than the feeding velocity (V₃) in which the driving roll (not shown) feeds the latch member continuums 140 to the lower blade roll 212. The circumferential velocity (V₁) of the upper blade roll 211 is substantially same as or slower than the moving velocity (V₄) of the flap continuum 130 that is continuously transported by the driving roll (not shown).

The lower blade roll 212 includes two stationary blades 212A that contacts with the blade 211A so as to cut the latch member continuum 140. The lower blade roll 212 is provided with a plurality of suction holes 212B configured to suck the latch member continuum 140.The lower blade roll 212 rotates in a direction opposite to that of the upper blade roll 211. As described above, the circumferential velocity (V₂) of the lower blade roll 212 is substantially same as the circumferential velocity (V₁) of the upper blade roll 211.

Between the lower blade roll 212 and the transcript roll 213, the transcript roll 213 transcripts (attaches) the latch members 42a and 42b cut by the upper blade roll 211 and the lower blade roll 212, on the flap continuum 130.

The circumferential velocity (V₅) of the transcript roll 213 is substantially same as the moving velocity (V₄) of the flap continuum 130. In other words, the circumferential velocity (V₅) of the transcript roll 213 is substantially same as, or faster than, the circumferential velocity (V₂) of the lower blade roll 212 and the circumferential velocity (V₁) of the upper blade roll 211.

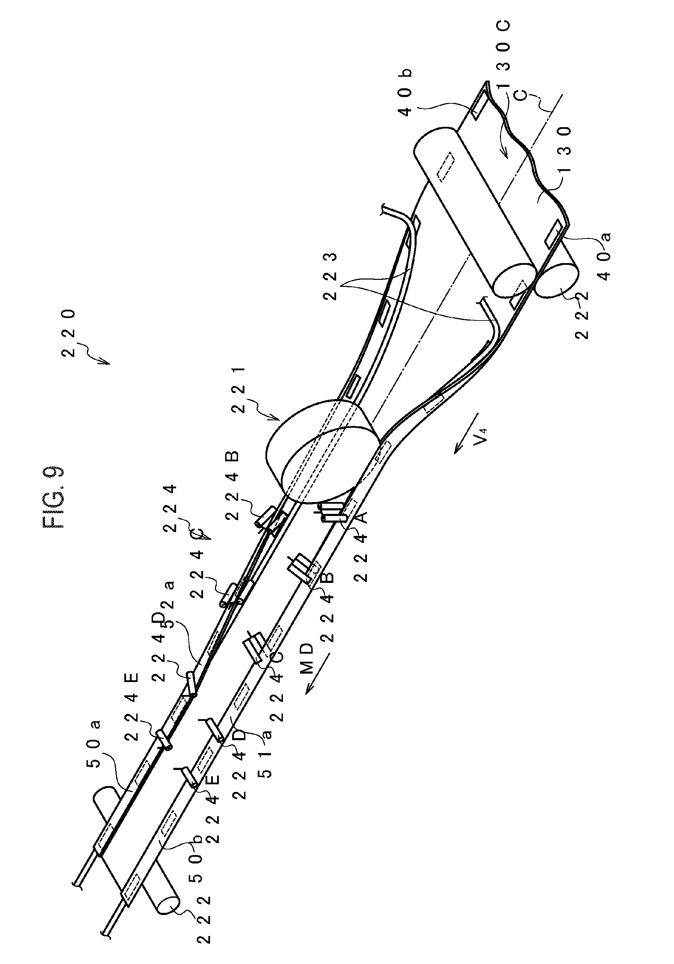

Next, the side edge folding back structure 220 according to the third embodiment will be described by referring to the accompanying drawings. Fig. 9 is a perspective view of the side edge folding back structure 220 according to the third embodiment of the present invention.

As shown in Fig. 9, the side edge folding back structure 220 folds back both of the side edge portions 50a and 50b placed on both sides of the flap continuum 130 toward the center line C.

The side edge folding back structure 220 is mainly provided with: a large diameter roll 221, a transporting roll 222, a folding center bar 223, and a guiding roll 224. Note that, the large diameter roll 221, the transporting roll 222, the folding center bar 223, and the guiding roll 224 are configured to rotate by using a shaft (not shown) as a center, respectively.

The large diameter roll 221 contact with the center region 130C of the flap continuum 130 (that is, center line C side with respect to the side edge portions 50a and 50b), and cause, along with the guiding roll 224 to be described later, the side edge portions 50a and 50b to stand.

The transporting roll 222 transports the flap continuum 130 in a substantially horizontal state. In other words, the flap continuum 130 is transported in a substantially horizontal state by the transporting roll 222.

The folding center bar 223 holds the folding back position, when the side edge portions 50a and 50b are folded back. The folding center bar 223 extends in the MD direction, and is placed in substantially parallel with the flap continuum 130 that is transported by the transporting roll 222.

The guiding roll 224 guides the side edge portions 50a and 50b so as to fold back the side edge portions 50a and 50b toward the center line C. The guiding roll 224 is provided with a plurality of guiding rolls 224A to 224E.

The side edge portions 50a and 50b are caused to stand and to be folded back via the folding center bar 223, gradually from the vicinity of the guiding roll 224A to the vicinity of the guiding roll 224E. In other words, the inclined angle of the side edge portions 50a and 50b with respect to the center region 130C of the flap continuum 130 becomes gradually small, from the vicinity of the guiding roll 224A to the vicinity of the guiding roll 224E.

In this regard, after the side edge portions 50a and 50 b are folded back by the side edge folding back structure 220, in order to prevent the flap continuum 130 from being turned over while being transported, an embossing step or the like may be performed on the folded back portions 51 a and 51 b, so that the side edge portions 50a and 50b can be temporarily bonded to the flap continuum 130. Here, the folded back portions 51a and 51b are referred to as portions formed by the side edge portions 50a and 50b being folded back to cause the flaps 30 to overlap.

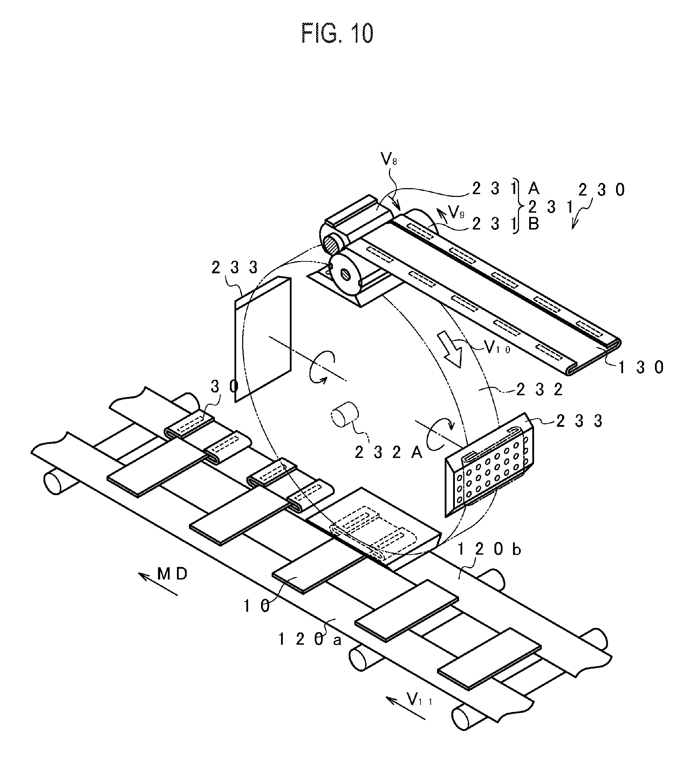

Next, the cutting and placing structure 230 according to the third embodiment will be described by referring to the accompanying drawings. Fig. 10 is a perspective view of the potential cut line forming structure 230 according to the third embodiment of the present invention.

As shown in Fig. 10, the cutting and placing structure 230 forms each of the flaps 30 by cutting the flap continuum 130, by the predetermined interval length in the CD direction. Further, the cutting and placing structure 230 places each of the flaps 30 on the rear waistline member continuum 120a so that the center line C is orthogonal to the MD direction of the main body line.

Specifically, the cutting and placing structure 230 is mainly provided with: a continuum cutting roll 231 and a rotating drum 232.

The continuum cutting roll 231 is provided with: a blade roll having a blade (not shown); and a stationary blade 231 B having a stationary blade (not shown). Note that, the circumferential velocity (V₈) of blade roll 231 A is substantially same as the circumferential velocity (V₉) of the stationary blade 231 B.

The rotating drum 232 includes a plurality of suction pads 233 configured to suck the flaps 30 cut by the blade roll 231 A and the stationary blade roll 231 B. The rotating drum 232 is configured to rotate by using the shaft 232A as a center so as to place the flaps 30, which are sucked by and attached to the suction pads 233, on the rear waistline member continuum 120a. Note that, the circumferential velocity (V₁₀) of rotating drum 232 is substantially same as the moving velocity (V₁₁) of the rear waistline member continuum 120a and the front waistline member continuum 120b.

While transporting the suction pads 233 to the upper side of the rear waistline member continuum 120a, the rotating drum 232 rotates the suction pads 233 by 90° by use of the rotating structure (not shown). In this manner, each of the flaps 30 is rotated by 90° and is inverted vertically by the cutting and rotating structure 230, and is bonded onto the rear waistline member continuum 120a.

### (Modified Example)

Hereinbelow, by referring to the accompanying drawings, a description will be given of a cutting and placing structure 230 according to the modified example of the third embodiment of the present invention. Fig. 9 is a perspective view of the cutting and placing structure 230 according to the modified example of the third embodiment of the present invention. Note that components having the same functions as the cutting and placing structure 230 of the third embodiment will be denoted by the same reference numerals, and descriptions will be mainly given for those different from the third embodiment.

In the manufacturing method for absorbent articles according to the present invention, on the main body line, each of the flaps 30 is placed on the rear waistline member continuum 120a so that the center line C is orthogonal to the main body line. In the above-described third embodiment, each of the flaps 30 is rotated by 90° and is inverted vertically, thereafter, each of the flaps 30 is bonded onto the rear waistline member continuum 120a. On the other hand, in the second embodiment, each of the flaps 30 is bonded onto the rear waistline member continuum 120a, without being rotated by 90° and is inverted vertically.

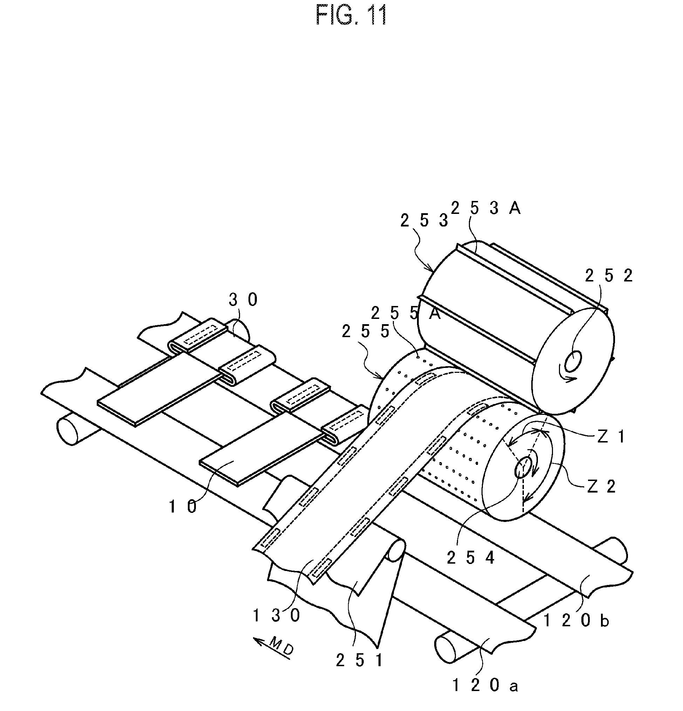

As shown in Fig. 11, the cutting and placing structure 250 according to the modified example cuts the flap continuum 130 by a predetermined interval length so as to form the flaps 30. Further, the cutting and placing structure 250 places the flaps 30 on the rear waistline member continuum 120a by use of a thermoplastic resin, without rotating the flaps 30 formed by the cutting.

Specifically, the cutting and placing structure 250 cuts the flap continuum 130, which is transported by an endless belt 251, by a predetermined interval length. Concurrently, the cutting and placing structure 250 attaches the flaps 30, which are formed by cutting the flap continuum 130, on the rear waistline member continuum 120a by use of a thermoplastic resin or the like.

The cutting and placing structure 250 is provided with: an upper blade roll 253 configured to rotate by using a shaft center 252 as a center; and a lower blade roll 255 configured to rotate by using a shaft center 254 as a center.

The upper blade roll 253 is provided with a plurality of blades 253A placed by a predetermined interval length on the outer peripheral surface thereof. Here, the plurality of blades 253A is placed in substantially parallel with the shaft center 252. The lower blade roll 255 is provided with: a suction hole 255A configured to suck the flaps 30 formed by cutting the flap continuum 130; and a stationary blade (not shown) configured to cut the flap continuum 130 with the blade 253A.

The peripheral surface of the lower blade roll 255 is provided with: a first zone Z1 that sucks and holds the flap continuum 130 with a predetermined suction force; and a second zone Z2 that sucks and holds the flaps 30 formed by cutting the flap continuum 130, with a suction force stronger than that of the first zone Z1.

When the flap continuum 130 passes through the first zone Z1, while slipping around the peripheral surface of the lower blade roll 255, the flap continuum 130 is sucked by the suction hole 255A formed on the first zone Z1 so as to be held by the peripheral surface of the lower blade roll 255. At this time, the flap continuum 130 is cut between the blade 253A and the stationary blade. Thereby, the flaps 30 are formed.

When the flaps 30 pass through the second zone Z2, without slipping around the peripheral surface of the lower blade roll 255, the flaps 30 are sucked by the suction hole 255A formed on the second zone Z2 so as to be held by the peripheral surface of the lower blade roll 255. Then, the flaps 30 approach the rear front waistline member continuum 120a by the rotation of the lower blade roll 255A so as to be attached thereon.

### [Other Embodiments]

It is a matter of course that orders of performing the steps in the above-described manufacturing method for the absorbent article is not limited to those described above as long as the absorbent article can be manufactured, and the steps to be performed can be appropriately selected in accordance with the intended purpose.

As described above, the present invention has been described in detail by using the above-described embodiments. However, it is obvious for a person skilled in the art that the present invention is not limited to the embodiments described in this specification. The present invention can be implemented as a modification and an amended embodiment without departing from the content and scope of the present invention which is defined by the description of the scope of claims. Accordingly, the description of the present invention is intended to give description as an example and does not have any meaning to limit the present invention.

This application is based upon and claims the benefit of priority from the prior Japanese Patent Application No. JP 2008-094064, filed on March 31, 2008, and Japanese Patent Application No. JP 2009-074773, filed on March 25, 2009, the entire contents of which are incorporated herein by reference.

### INDUSTRIAL APPLICABILITY

As mentioned above, according to the present invention, there can be provided a manufacturing method for absorbent articles being able to realize a stable transportation, and to reduce a material loss in forming side flaps without requiring any complex step.

## Claims

1. A manufacturing method for absorbent articles each including: front waistline members, rear waistline members, and crotch members, the crotch members connecting each of the front waistline members and each of the rear waistline members, comprising:
placing latch members that can be attached to and locked in predetermined regions, on both sides of a vicinal region of a center line extending along a moving direction of a flap line, on a flap continuum having a long shape and being continuously transported on the flap line;
forming flaps on the flap line, by cutting the flap continuum in a direction orthogonal to the moving direction of the flap line; and
placing each of the flaps, on a main body line, on a front waistline member continuum or on a rear waistline member continuum, so that the center line is orthogonal to a moving direction of the main body line, wherein
on the flap line, both side edge portions of the flap continuum are folded back toward the center line.

2. The manufacturing method for the absorbent articles according to claim 1, further comprising:
cutting the front waistline member continuum and the rear waistline member continuum, along a direction crossing the moving direction of the main body line, on the main body line by a predetermined interval length, wherein
a width of each of the flaps in a direction crossing the center line is shorter than the predetermined interval length.

3. The manufacturing method for the absorbent articles according to claim 1, wherein,
in placing the latch members, the latch members are placed on the both side edge portions of the flap continuum, and
the both side edge portions of the flap continuum are folded back toward the center line.

4. The manufacturing method for the absorbent articles according to claim 3, wherein
the both side edge portions of the flap continuum are folded back toward the center line, and the latch members placed on the both edge portions are attached to and locked on the flap continuum.

5. The manufacturing method for the absorbent articles according to claim 3, further comprising:
forming cut-out portions each having predetermined shapes, in a part of the both side edge portions.

6. The manufacturing method for the absorbent articles according to claim 1, wherein,
in placing the latch members on the flap continuum, the latch members are placed on both sides of a vicinal region of the center line that extends along the moving direction of the flap line, and further comprising:
forming a potential cut line that halves the flap continuum along the center line.

7. The manufacturing method for the absorbent articles according to claim 6, further comprising:
forming cut-out portions each having predetermined shapes, in a part of the vicinal region of the center line.

8. The manufacturing method for the absorbent articles according to claim 1, wherein,
in placing the flaps, the flaps are bonded onto the front waistline member continuum or the rear waistline member continuum.

9. The manufacturing method for the absorbent articles according to claim 1, further comprising:
placing the crotch members so that each of the crotch members are apart from each other in the moving direction of the main body line, between the front waistline member continuum and the rear waistline member continuum both having a long shape and being transported continuously on the main body line.

10. The manufacturing method for the absorbent articles according to claim 1, wherein,
in placing the flaps, the flaps are placed so as to cover edge portions of the crotch members.

11. The manufacturing method for the absorbent articles according to claim 2, wherein,
in the cutting, the front waistline member continuum and the rear waistline member continuum are cut so that each lengths of the front waistline members in a width direction of the absorbent articles is equal to each lengths of the rear waistline members in a width direction of the absorbent articles.

12. A manufacturing apparatus for absorbent articles each including front waistline members, rear waistline members, and crotch members, the crotch members connecting each of the front waistline members and each of the rear waistline members, comprising:
a latch member placing structure configured to place latch members that can be attached to and locked in predetermined regions, on both sides of a vicinal region of a center line extending along a moving direction of a flap line, on a flap continuum having a long shape and being continuously transported on the flap line;
a folding back structure configured to fold back both side edge portions of the flap continuum toward the center line; and
a cutting and placing structure configured to form flaps by cutting the flap continuum in a direction orthogonal to the moving direction of the flap line, and to place each of the flaps on a front waistline member continuum or on a rear waistline member continuum, so that the center line is orthogonal to a moving direction of a main body line.
